# EUROPEAN PATENT SPECIFICATION

(11) **EP 0 933 359 B1**
(45) Date of publication and mention of the grant of the patent: **18.12.2002**
(21) Application number: 98105751.6
(22) Date of filing: 30.03.1998
(51) Int. Cl.: C07C 403/24

(54) **Method of obtaining a composition containing 9-cis beta-carotene in high-purity**
Verfahren zur Herstellung einer 9-cis-beta-Carotin in hoher Reinheit enthaltenden Zusammensetzung
Procédé pour la préparation d' une composition contenant 9-cis-bêta-carotène de pureté élevée

(30) Priority: 26.12.1997 JP 35979897
(43) Date of publication of application: 04.08.1999
(73) Proprietor: Institute of Applied Biochemistry, Kani-gun, Gifu-ken 505-0100 (JP)
(72) Inventor: Suzuki, Takehiko, Kasugai-shi, Aichi-ken, 486-0926 (JP); Ohishi, Nobuko, Inuyama-shi, Aichi-ken, 484-0073 (JP); Yagi, Kunio, Nagoya, Aichi-ken, 465-0084 (JP)
(74) Representative: VOSSIUS & PARTNER

(56) References cited:
- US-A- 5 310 554
- T. SUZUKI ET AL: BIOCHEM. MOL. BIOL. INT., vol. 39, no. 6, 1996, pages 1077-1084, XP002043882

## Description

The present invention relates to a method of obtaining a composition containing 9-cis β -carotene in high-purity.

β -Carotene is one of carotenoids contained in various colored vegetables and so on and has been well known as a substance which changes to vitamin A in vivo to show high biological activity. Further, β -carotene has high expectation as an ingredient for medicines, since it shows an anti-tumor activity and has the ability of eliminating active oxygen.
Natural β -carotene is mainly of all-trans type but various geometrical isomers thereof are present, and in the recent years, there has been great interest in the activities of the isomers, since such isomers have been detected in human tissue, in addition to all-trans β -carotene.
Especially, 9-cis β -carotene has become focus of such an attention, among the isomers. Since 9-cis β -carotene is produced and accumulates in relatively large amount in green algae belonging to microalgae, Dunaliella, extractions of the substance from such a source have been tried [USP 5,310,554; USP 5,612,485; and Yuan et al., "Yaowu Shengwu Jishu", Vol. 3, pages 34 - 39 (1996) and so on].

However, almost all of the conventional methods of obtaining the composition containing 9-cis β -carotene utilize column chromatography and thus are not suitable for industrial scale production of the composition. The inventors have also tried to extract 9-cis β -carotene from Dunaliella bardawil by using hexane and obtained a solid composition containing 9-cis β-carotene, but the content of 9-cis β-carotene therein varies remarkably in each experiment, so that they applied many complicated procedures including column chromatography to obtain fine needle-shaped 9-cis β -carotene as follows: removing the solid materials in hexane to obtain a filtrate, subjecting the filtrate to silica gel chromatography, concentrating a carotene-rich fraction to obtain foamy solids, sonicating the solids in ethanol to obtain orange particles, dissolving the particles in chloroform, subjecting the resulting solution to ODS open column chromatography, concentrating a carotene-rich fraction to obtain an orange powder, subjecting the powder to preparative high-performance liquid chromatography (HPLC), and then concentrating the HPLC fraction which contains 9-cis β-carotene to cause precipitation thereof as the crystals [Susuki et al., "Biochem. Mol. Biol. Int.", Vol. 39, pages 1077 - 1084 (1996)].

Methods of obtaining 9-cis β -carotene utilizing no column chromatography have also been proposed, but all reports on such proposals have reported that the composition obtained is an oily substance, in spite of the fact that 9-cis β-carotene, inherently, is a solid substance having a melting point of 122 - 123 °C [Polgar et al., "J. Amer. Chem. Soc.", Vol. **64,** pages 1856 - 1861 (1942)]. This means that each of the compositions contains impurities other than 9-cis β -carotene, to some extent. In reports, it has often been mentioned that "high-purity 9-cis β -carotene" was obtained by referring to its ratio to total carotene, but these reports are questionable in that impurities other than carotenes have not been taken into consideration and this becomes a greet problem if the composition shall be actually used as an ingredient for medicines.

An object of the invention, therefore, is to provide a method of obtaining a composition containing 9-cis β -carotene, which has not only a high ratio of 9-cis β -carotene to total carotene, but also only a small amount of impurities other than carotenes and which can be referred to as a "high-purity 9-cis β-carotene", and, which is applicable to an industrial scale production thereof.

The invention is based on the finding that a powdery or crystalline composition containing 9-cis β -carotene can be obtained by washing with ethanol a dry powder of an alga belonging to Dunaliella, and then treating with hexane in a stepwise manner. Analysis of the composition shows a high percentage of 9-cis β-carotene. The procedures are similar to those disclosed in said literature of "Biochem. Mol. Biol. Int.", Vol. 39, pages 1077 - 1084 (1996), but greatly different in that in the method of the literature, the final treatment by hexane is carried out to cause precipitation of remaining all-trans β -carotene, whereas in the method according to the invention, the treatment is carried out to cause precipitation of not all-trans β -carotene but 9-cis β -carotene.
The method of obtaining the composition containing 9-cis β-carotene in high-purity according to the invention comprises a first step consisting essentially of washing with ethanol a dry powder of an alga belonging to Dunaliella, adding hexane to, and stirring the washed powder, which is then subjected to filtration, and concentrating the filtrate to obtain a semi-solid concentrate; a second step consisting essentially of adding hexane to said semi-solid concentrate in an amount of 10 - 18ml of hexane per 1g of said semi-solid concentrate to stir the mixture of hexane and said semi-solid concentrate, which is then subjected to filtration, and concentrating the filtrate to obtain an oily concentrate; and a third step consisting essentially of adding hexane to said oily concentrate in an amount of 2 - 4.5ml of hexane per 1g of said oily concentrate to dissolve the oily concentrate, leaving the solution, to stand, to cause a separation of solids, obtaining, washing, and drying the solids. The composition obtained acording to the invention is is characterized by a rubiginous powder or crystals containing 9-cis β -carotene in an amount of 75% or more to total amount of the composition.

As a raw material for carrying out the invention, a micro green alga belonging to Dunaliella is used. Since it has been known that the algae belonging to Dunaliella produce 9-cis β -carotene in large amounts to accumulate the same therein and more particularly, 9-cis β -carotene accumulates in large amounts in Dunaliella bardawil and Dunaliella salina, it is preferable to select these algae. For instance, Dunaliella bardawil usually contains 9-cis β -carotene and all-trans β-carotene in a ratio of about 1 : 1.

The first step of the method according to the invention is a procedure for washing the alga and extracting carotenes. Firstly, ethanol is added to the dry powder of the alga and the mixture is stirred to dissolve ethanol-soluble substances as much as possible and remove the substances by filtration. The stirring operation may be carried out at room temperature and there is no special limitation in the amount of ethanol to be added and the stirring period. No substantial loss of 9-cis β -carotene will result in this washing treatment, since the solubility of β-carotenes in ethanol is quite low.
The washing operation with ethanol is very important. Because impurities and carotenes interact with each other in an adsorptional manner, when the impurities are not sufficiently removed by this washing treatment, the composition containing 9-cis β-carotene cannot finally be obtained as solids. Therefore, it is preferable to carry out the washing operation repeatedly.
After having obtained ethanol-insoluble substance by filtration, hexane is added thereto and the mixture is stirred to cause elution of carotenes. The carotene containing solution is filtered and the filtrate is concentrated to obtain a semi-solid substance (hereinafter referred to as "concentrate A").

The second step is a procedure for separating and removing all-trans β -carotene from the concentrate A obtained by the first step. The procedures utilize the difference in solubility of 9-cis β -carotene and all-trans β -carotene in hexane, namely the solubility of the former is 2g/100ml (24°C) and the latter is 0.11g/100ml (30°C), to cause separation of all-trans β -carotene for removing the same.
In connection with the procedures, it is necessary to determine the content of each carotene in the concentrate A, and the contents can be measured by HPLC. By this measurement, an optimum amount of hexane to be added to the concentrate A can be calculated from solubility of the carotenes in hexane and the carotene contents in the concentrate A. However, the solubility of 9-cis β-carotene and all-trans β -carotene is greatly influenced by impurities in the concentrate A and thus in many instances the actual optimum amount of hexane to be added for separating all-trans β -carotene from 9-cis β -carotene does not coincide with the calculated amount.
According to the present invention, it has been found that it is suitable to add hexane in an amount of 10 - 18ml per 1g of the concentrate A, as stated before. Thus, hexane is added to the concentrate A in such an amount and the mixture is stirred for causing separation or precipitation of all-trans β -carotene which is removed by, for instance, filtration.

In case the amount of hexane to be added to the concentrate A is less than said range, namely the amount is less than 10ml per 1g of the concentrate A, 9-cis β -carotene is apt to be precipitated together with all-trans β -carotene to cause loss of the former. In case the amount of hexane is more than 18ml per 1g of the concentrate A, the amount of all-trans β-carotene remaining in the hexane increases, which makes it difficult to obtain a final composition containing 9-cis β-carotene in high-purity.
The stirring operation may be carried out at room temperature, but it is preferable to select a lower temperature condition, for instance, a temperature of about 0°C for separating all-trans β -carotene with good efficiency.
Thereafter, all-trans β-carotene is separated by, for instance, filtration and the filtrate is concentrated to obtain a viscous oily substance (hereinafter referred to as "concentrate B").

The third step is a procedure for separating from the concentrate B the composition containing 9-cis β -carotene, as a solid. The composition can be separated by adding hexane to the concentrate B obtained by the second step, stirring the same and then leaving it to stand. The separated solids are collected by filtration, washed with ethanol and then dried to give the desired composition containing 9-cis β -carotene in high-purity, in the form of a powder or fine crystals.
In the concentrate B obtained by the second step, there are some impurities other than the carotenes. The impurities at this stage easily dissolve in hexane and thus, for separating 9-cis β-carotene, it is preferable to use hexane in an amount as small as possible.
According to the present invention, the optimum amount of hexane for separating the composition containing 9-cis β -carotene is 2 - 4.5ml per 1g of the concentrate B, as stated before. Namely, when hexane is added to the concentrate B in such a ratio to dissolve the same and leaving the solution to stand, the composition containing 9-cis β-carotene separates, as a solid.

In case the amount of hexane to be added to the concentrate B is less than said range, namely the amount is less than 2ml per 1g of the concentrate B, the interactions of 9-cis β-carotene with the impurities make it difficult to separate 9-cis β -carotene, as a solid and even if the separation of 9-cis β-carotene is possible, the impurities are apt to be taken into the separated solids resulting in a lower purity of the final composition. In case the amount of hexane is more than 4.5ml per 1g of the concentrate B, the amount of 9-cis β -carotene remaining in hexane increases to cause a decrease in the yield of the desired composition.
The operation may be carried out at room temperature, but it is preferable to select a lower temperature condition, for instance, a temperature of about -20°C for separating 9-cis **β**-carotene with good efficiency.
The separated solids are collected by filtration, washed with ethanol and then dried to afford the desired composition containing 9-cis β -carotene in high-purity, in the form of a powder or fine crystals.
The resulting composition contains 9-cis β -carotene in an amount of 80% or more to total carotene and 75% or more to the total amount containing impurities.

The invention will now be further explained with reference to a Production Example and Test Example which shall refer to a drawing of chromatogram showing results of HPLC made on a composition containing 9-cis β -carotene according to the invention.

### Example (Production of composition containing 9-cis β -carotene)

To a dry powder of Dunaliella bardawil (2000g), ethanol (8000ml) was added, stirred for 15 minutes at room temperature, and then filtered. Ethanol (8000ml) was again added to solids obtained by filtration. The mixture was stirred for 15 minutes at room temperature, and then filtered to obtain the washed algal powder. To the washed powder, hexane (18000ml) was added. The mixture was stirred for 30 minutes at room temperature, and then filtered to obtain a filtrate. The filtrate was concentrated to obtain a blackish brown semi-solid substance (98.46g). To the semi-solid substance, hexane (1477ml, corresponding to 15ml per 1g of the semi-solid substance) was added. The mixture was stirred for one hour at 0°C, and then filtered to obtain a filtrate. The filtrate was concentrated to obtain a viscous oily substance (59.59g) which has a glossy blackish brown color. To the oily concentrate, hexane (209ml, corresponding to 3.5ml per 1g of the oily concentrate) was added to immediately dissolve the same. The mixture was then left to stand over night at -20°C. Separated solids were collected by filtration, washed with ethanol, and dried to obtain 31.66g of a desired composition containing 9-cis β -carotene in high-purity, as rubiginous powder.

### Test Example (HPLC analysis)

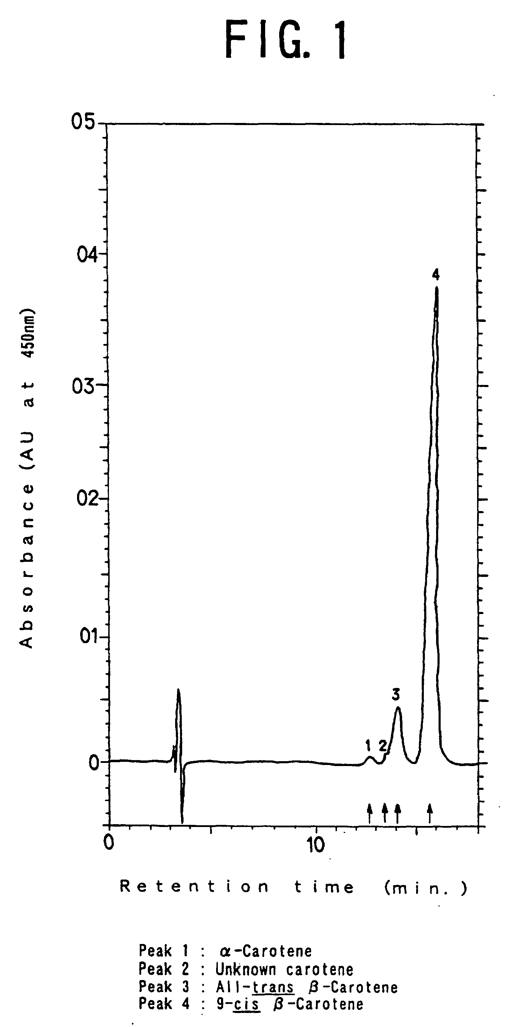

The composition obtained by said Example was subjected to HPLC. Analytical conditions therefore were as follows.
Detector : Detector with a photodiode array (Type MCPD-3600 manufactured by Tosoh Corp.),
Detection wave-length : 450nm,
Guard column : TSK guardgel ODS-120A (3.2mm i.d. x 15mm, manufactured by Tosoh Corp.),
Analytical column : Vydac 201TP54 (4.6mm i.d. x 250mm, manufactured by The Separations Group, Hesperia, CA),
Column temperature : 27 ± 0.1°C, and
Mobile phase : : Mixture of methanol and chloroform (95 : 5, V/V) containing 10mM ammonium acetate,
Flow rate : 1ml/min.
A chromatogram under such analytical conditions is shown in Fig. 1. Calculation of the ratio of 9-cis β-carotene to total carotene based on the analytical results was 86.8% showing that the composition obtained by the invention contains 9-cis β -carotene in high-purity.
It was found that the peak area of 9-cis β -carotene part in this analysis (measured value, namely AU x Δ min) is 0.0190.
Purified 9-cis β -carotene was obtained by refining and isolating the same through HPLC and then subjecting it to crystallization. The purified 9-cis β -carotene in the form of needle crystals was analyzed under conditions as given above to show that the peak area is 0.0225. Therefore, it was found that the content of 9-cis β -carotene in the total amount of the composition obtained by the method according to the invention is 84.4%.

## Claims

1. A method of obtaining a composition containing 9-cis β-carotene in high-purity, which comprises a first step consisting essentially of washing with ethanol a dry powder of an alga belonging to Dunaliella, adding hexane to, and stirring the washed powder, which is then subjected to filtration, and concentrating the filtrate to obtain a semi-solid concentrate; a second step consisting essentially of adding hexane to said semi-solid concentrate in an amount of 10 - 18ml of hexane per 1g of said semi-solid concentrate to stir the mixture of hexane and said semi-solid concentrate, which is then subjected to filtration, and concentrating the filtrate to obtain an oily concentrate; and a third step consisting essentially, of adding hexane to said oily concentrate in an amount of 2 - 4.5ml of hexane per 1g of said oily concentrate to dissolve the oily concentrate, leaving the solution, to stand, to cause a separation of solids, separating, washing, and drying the solids.

2. A method of obtaining a composition as claimed in Claim 1, wherein said alga belonging to Dunaliella is selected from the group consisting of Dunaliella bardawil and Dunaliella salina.

3. A method of obtaining a composition as claimed in Claim 1, wherein the stirring operation in said second step is carried out under lower temperature condition.

4. A method of obtaining a composition as claimed in Claim 3, wherein said stirring operation is carried out at 0°C.

5. A method of obtaining a composition as claimed in Claim 1, wherein the said solution in the third step is left to stand under lower temperature condition.

6. A method of obtaining a composition as claimed in Claim 5, wherein the temperature of said solution left to stand is -20°C.

## Patentansprüche

1. Verfahren zur Herstellung einer 9-cis-β-Carotin in hoher Reinheit enthaltenden Zusammensetzung, umfassend einen ersten Schritt, im wesentlichen bestehend aus dem Waschen eines Trockenpulvers einer zu Dunaliella gehörenden Alge mit Ethanol, dem Zusetzen von Hexan und dem Rühren des gewaschenen Pulvers, welches dann einer Filtration unterworfen wird, und der Konzentrierung des Filtrats, um ein halbfestes Konzentrat zu erhalten; einen zweiten Schritt, im wesentlichen bestehend aus dem Zusetzen von Hexan zu dem halbfesten Konzentrat in einer Konzentration von 10 - 18 ml Hexan pro einem Gramm des halbfesten Konzentrats, dem Rühren des Gemisches aus Hexan und dem halbfesten Konzentrat, welches dann einer Filtration unterworfen wird, und dem Konzentrieren des Filtrats, wobei ein öliges Konzentrat erhalten wird; und einem dritten Schritt, im wesentlichen bestehend aus dem Zusetzen von Hexan zu dem öligen Konzentrat in einer Menge von 2 - 4,5 ml Hexan pro einem Gramm des öligen Konzentrats, um das ölige Konzentrat zu lösen, dem Stehenlassen der Lösung, um eine Abtrennung der Feststoffe zu erreichen, dem Abtrennen, dem Waschen, und dem Trocknen der Feststoffe.

2. Verfahren zur Herstellung einer Zusammensetzung nach Anspruch 1, wobei die zu Dunaliella gehörende Alge ausgewählt ist aus der Gruppe bestehend aus Dunaliella bardawil und Dunaliella salina.

3. Verfahren zur Herstellung einer Zusammensetzung nach Anspruch 1, wobei der Rührprozess im zweiten Schritt unter niedrigerer Temperaturbedingung durchgeführt wird.

4. Verfahren zur Herstellung einer Zusammensetzung nach Anspruch 3, wobei der Rührprozess bei 0° C durchgeführt wird.

5. Verfahren zur Herstellung einer Zusammensetzung nach Anspruch 1, wobei die Lösung im dritten Schritt unter niedrigerer Temperaturbedingung stehen gelassen wird.

6. Verfahren zur Herstellung einer Zusammensetzung nach Anspruch 5, wobei die Temperatur beim Stehenlassen der Lösung - 20° C ist.

## Revendications

1. Méthode d'obtention d'une composition contenant du 9-cis-β-carotène avec une haute pureté, qui comprend une première étape consistant essentiellement à laver à l'éthanol une poudre sèche d'une algue Dunaliella, à ajouter de l'hexane, et à mélanger la poudre lavée qui est ensuite soumise à une filtration, et à concentrer le filtrat pour obtenir un concentré semi-solide ; une deuxième étape consistant essentiellement à ajouter de l'hexane audit concentré semi-solide à raison de 10 - 18 ml d'hexane par 1 g de concentré semi-solide, à mélanger le mélange d'hexane et dudit concentré semi-solide qui est ensuite soumis à une filtration, et à concentrer le filtrat pour obtenir un concentré huileux; et une troisième étape consistant essentiellement à ajouter de l'hexane audit concentré huileux à raison de 2 - 4,5 ml d'hexane par 1 g dudit concentré huileux pour dissoudre le concentré huileux, à laisser reposer la solution pour causer la séparation des solides, à séparer, à laver, et à sécher les solides.

2. Méthode d'obtention d'une composition selon la revendication 1, dans laquelle ladite algue Dunaliella est sélectionnée dans le groupe constitué de Dunaliella bardawil et Dunaliella salina.

3. Méthode d'obtention d'une composition selon la revendication 1, dans laquelle l'opération de mélange dans ladite deuxième étape est effectuée dans des conditions de température plus basse.

4. Méthode d'obtention d'une composition selon la revendication 3, dans laquelle ladite étape de mélange est effectuée à 0°C.

5. Méthode d'obtention d'une composition selon la revendication 1, dans laquelle ladite solution dans la troisième étape est laissée à reposer dans des conditions de température plus basse.

6. Méthode d'obtention d'une composition selon la revendication 5, dans laquelle la température de ladite solution laissée à reposer est -20°C.
